# EUROPEAN PATENT SPECIFICATION

(11) **EP 4 483 749 B1**
(45) Date of publication and mention of the grant of the patent: **11.03.2026**
(21) Application number: 24184423.2
(22) Date of filing: 25.06.2024
(51) Int. Cl.: A45D 34/04, A45D 40/26, A45D 34/00, A45D 40/00

(54) **APPLICATOR FOR ATTACHING PATCH TO THE SKIN**
APPLIKATOR ZUR ANBRINGUNG EINES PFLASTERS AUF DER HAUT
APPLICATEUR POUR FIXER UN TIMBRE À LA PEAU

(30) Priority: 30.06.2023 KR 20230084671
(43) Date of publication of application: 01.01.2025
(73) Proprietor: Amorepacific Corporation, Seoul 04386 (KR)
(72) Inventor: Oh, Juwon, 17074 Yongin-si (KR)
(74) Representative: Schön, Christoph

(56) References cited:
- WO-A1-2020/260056
- JP-A- 2007 007 345
- US-A- 3 516 423

## Description

### CROSS-REFERENCE TO RELATED APPLICATION(S)

### TECHNICAL FIELD

The present disclosure relates to an applicator for attaching a patch to the skin.

### BACKGROUND

In general, skin care such as exfoliation, moisturizing or nourishing is performed by applying various cosmetic products such as various skin lotions, creams, essences, etc. evenly to provide nutrients to the skin. However, the cosmetic products, which is intended for application, is inconvenient in use, so recently, patchable cosmetic products, which can be used simply by attaching it to the skin, has been provided.

The patchable cosmetic products may be provided in the form of a thin film, containing cosmetics on one side, and may be provided in a form that can be attached to the skin.

This patchable cosmetic products were generally used by the user manually detaching it from a release sheet and attaching it to the skin without a separate tool. However, there are problems with removing the patchable cosmetic product by hand in this way, such as foreign objects sticking to the patchable cosmetic product during the removal process, or the patchable cosmetic product being folded during the attachment process.

In addition, since it is difficult to accurately distribute pressure to the patchable cosmetic product at the time of attaching the patchable cosmetic product to the skin, there is a problem that accurate adhesion is not achieved and folding or lifting phenomenon occurs.
[Prior Art] (Patent Document 1) Korean Patent No. 10-1528741

JP 2007 007345 A discloses a sheet sticking device comprising a sheet mount part at least in a part of a pushing part, wherein when a sheet having adhesion at least on a surface to be applied to the skin of a user is pushed against the skin of the user by the pushing part with the sheet mounted on the mount part, the sheet can be stuck to the skin and after the sticking, the sheet is removed therefrom.

### SUMMARY

An applicator for attaching a patch to the skin according to the present invention is defined in appended claim 1 and was proposed to solve the above problem, and provides an applicator, which allows the patch to be easily removed from the release sheet and to be attached to the skin simply and effectively.

### BRIEF DESCRIPTION OF DRAWINGS

FIG. 1 is a perspective view showing the overall appearance of an applicator for attaching a patch to the skin according to the present invention.
FIG. 2 is a perspective view in enlargement of the coupling unit portion of FIG. 1.
FIG. 3 is a front view, in enlargement of a coupling unit modified according to another embodiment of the present disclosure.
FIG. 4 is a front view, in enlargement of a coupling unit according to another embodiment of the present disclosure.
FIG. 5 is a top plan view separately showing the appearance of a patch-attaching unit in which an adsorption groove is formed according to the present invention.
FIGS. 6A and 6B are showing the transformation process of the supporting unit and patch-attaching unit according to one embodiment of the present disclosure.
FIGS. 7A, 7B, and 7C are showing the process of attaching and detaching a patch to an applicator according to an embodiment of the present disclosure.

### DETAILED DESCRIPTION OF EXEMPLARY EMBODIMENTS

Hereinafter, preferred embodiments of the present disclosure are described in detail with reference to the attached drawings. Note that in the accompanying drawings, identical components are denoted with the same symbols as possible. We will also omit a detailed description of the announcement features and configurations that would obscure the gist of the present disclosure. For the same reason, some components are exaggerated, omitted, or schematically depicted in the accompanying drawings.

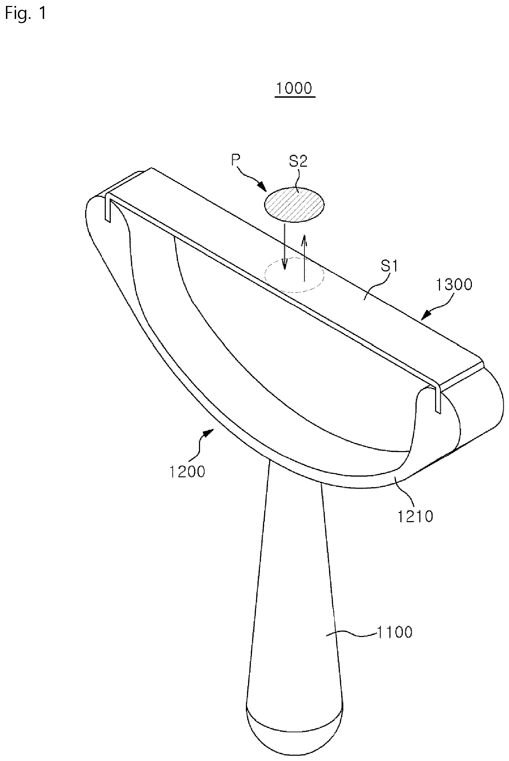

FIG. 1 is a perspective view, showing the overall appearance of an applicator for attaching a patch to the skin according to one embodiment of the present disclosure.

Hereinafter, referring to FIG. 1, the overall configuration of the applicator 1000 according to the present invention which is defined in appended claim 1 will be described. The applicator 1000 according to one embodiment of the present disclosure may include a handle 1100, and includes a supporting unit 1200 and a patch-attaching unit 1300.

The applicator 1000 is a part that the user grips, and may be provided so that the user may use the applicator 1000 after gripping it. Specifically, the handle 1100 may be formed in a shape that elongates in one direction. In this embodiment, it is shown as an elongated shape extending upward and downward relative to FIG. 1. The handle 1100 may be more convex on the bottom than on the top. The handle 1100 may be circular in the cross-section. The handle 1100 may be omitted or formed as part of the supporting unit 1200 in accordance with the embodiment. In addition, it may be formed to be indistinguishable from the supporting unit 1200 in appearance, and in this case, the part that the user arbitrarily grips can be understood as playing the role of the handle 1100.

The supporting unit 1200 is a component that is elastically deformed in one direction and restored in the other direction, and may elastically support the patch-attaching unit 1300. For example, the supporting unit 1200 may be formed as a curve shape with a preset curvature. Specifically, the supporting unit 1200 may have an arc-shape or a 'U'-shaped cross-section, and the curved line may transform into a wider open shape and then restored to the original state.

As one example, the supporting unit 1200 may have a shape extending from the handle 1100. The supporting unit 1200 may be formed integrally with the handle 1100 or may be formed separately and joined to the supporting unit 1200 by various methods, such as glued, screwed, fused, etc. in accordance with the embodiment. This allows the supporting unit 1200 has a shape connected to the handle 1100.

The supporting unit 1200 may be formed by extending from an end on one side of the handle 1100. In FIG. 1, the supporting unit 1200 is illustrated to be extending from an upper end of the handle 1100 as an example.

As described above, the supporting unit 1200 may be provided to be elastically deformable. The supporting unit 1200 may be formed from a material having a first elastic modulus E1. In other words, the supporting unit 1200 may have the first elastic modulus E1. Accordingly, the supporting unit 1200 is elastically deformed when an external force is exerted.

The supporting unit 1200 may include an extending unit 1210. The supporting unit 1200 may include the extending unit 1210 formed by extending on both sides from the handle 1100. Herein, the extending unit 1210 may have a shape extending from the handle 1100 to curve shape with a predetermined curvature. In addition, the extending unit 1210 extending on both sides from the handle 1100 may have a symmetrical shape with respect to the handle 1100. Through this, when an external force from above is applied to the supporting unit 1200, the supporting unit 1200 may be elastically deformed to spread to both sides, and in case a patch P is attached to the center of the patch-attaching unit 1300, the force may be evenly distributed to both sides of the supporting unit 1200.

In case the supporting unit 1200 is formed by extending on both sides from the handle 1100, the supporting unit 1200 may support the patch-attaching unit 1300 on both sides. With the upper end of the handle 1100 as a center, an included angle between the extending units 1210 of the supporting unit 1200 may be less than 180 degrees. For example, the included angle between the extending units 1210 may be 120 degrees, but is not limited thereto.

The applicator 1000 with such a shape may have a structure in which the handle 1100 is formed on one side and the patch-attaching unit 1300 on the other side relative to the supporting unit 1200.

The supporting unit 1200 may be concavely formed toward the patch-attaching unit 1300. In other words, the supporting unit 1200 may be formed convexly toward the handle 1100. That is, the supporting unit 1200 and the patch-attaching unit 1300 may form a 'D' shape as a whole when viewed from the side. In this case, even if the patch-attaching unit 1300 is deformed by the flexion of an amorphous face, the deformation of the patch-attaching unit 1300 may be induced into the concave space of the supporting unit 1200, allowing the user to use the applicator 1000 conveniently.

The patch-attaching unit 1300 is supported by the supporting unit 1200. One side of the patch-attaching unit 1300 may be disposed facing downwardly toward the supporting unit 1200. A first adhesive surface S1 is formed at the other side to the patch-attaching unit 1300. The patch P may be temporarily adhered to the first adhesive surface S1 of the patch-attaching unit 1300.

The patch-attaching unit 1300 may be formed from a material having adhesive properties, may have an adhesive applied to the portion corresponding to the first adhesive surface S1, or may be provided with an adhesive film attached. As one example, the patch-attaching unit 1300 may be an adhesive sheet. In addition, the patch-attaching unit 1300 may be made of a material with elasticity so that it may be elastically deformed in response to the curves of the face of a user by using the applicator 1000 and then restored to its original state. Herein, when the supporting unit 1200 is elastically deformed and tensioned, the patch-attaching unit 1300 is also tensioned together, and when the supporting unit 1200 is restored to the original state, the patch-attaching unit 1300 may also be restored to the original state.

As one example, the patch-attaching unit 1300 may be a silicon sheet having an adhesive film attached to one side thereof.

The patch P may have a second adhesive surface S2 on one side that may be adhered to the skin. The second adhesive surface S2 may contain cosmetic materials, etc. The other side of the patch P may temporarily stick to the first adhesive surface S1 of the patch-attaching unit 1300.

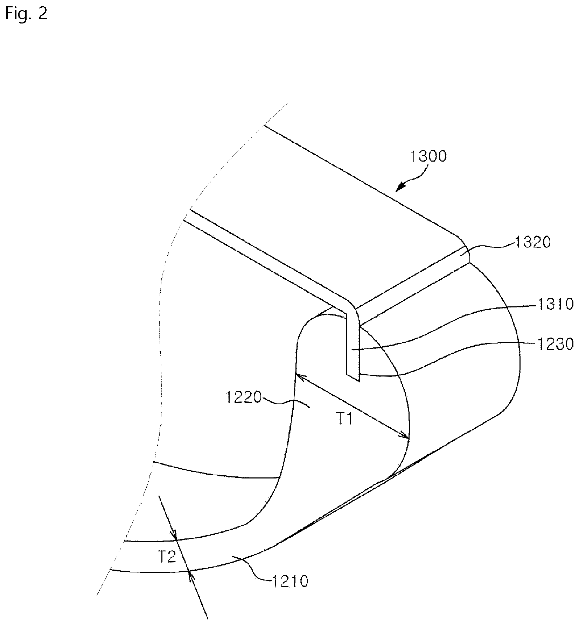

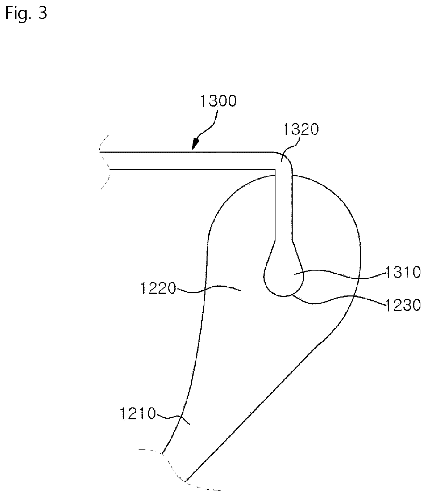

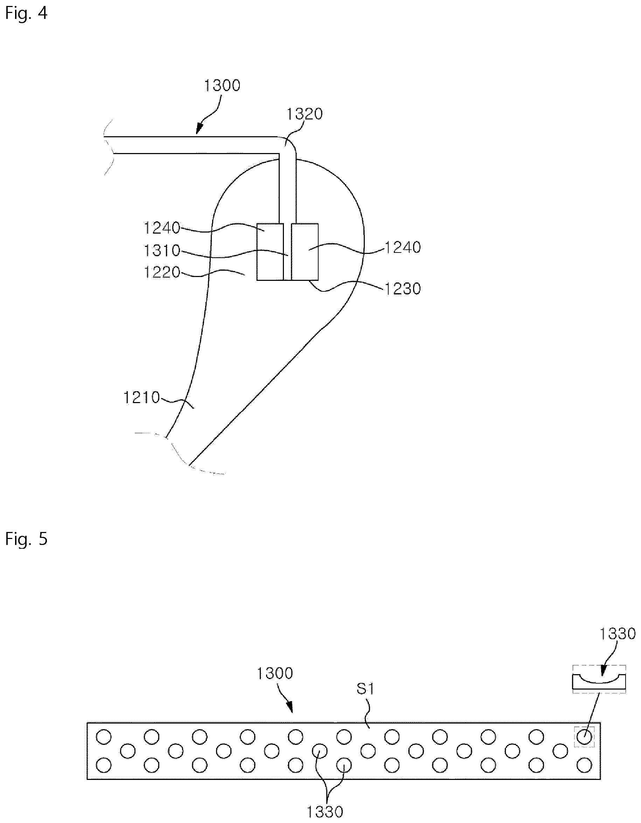

FIG. 2 is a perspective view, in enlargement of the coupling unit portion of FIG. 1, FIG. 3 is a front view, in enlargement of the modified coupling unit according to another embodiment of the present disclosure, and FIG. 4 is a front view in enlargement of the coupling unit according to another embodiment of the present disclosure.

Hereinafter, referring to FIG. 2 to FIG. 4, the supporting unit 1200 and the patch-attaching unit 1300 will be described in more detail in accordance with the embodiments of the present disclosure.

The coupling unit 1220 may be formed in the supporting unit 1200. The patch-attaching unit 1300 may be coupled to the coupling unit 1220. In the coupling unit 1220, the two ends 1310 of the patch-attaching unit 1300 may be coupled.

The coupling unit 1220 may be a portion of the extending unit 1210. The coupling unit 1220 may be formed on the outer side or upside of the end of the extending unit 1210, respectively.

The thickness T1 of the coupling unit 1220 may be formed thicker than the thickness T2 of the remaining portion of the supporting unit 1200. In this case, where a predetermined amount of external force is exerted to the supporting unit 1200, the amount of elastic deformation of the coupling unit 1220 is less than the amount of elastic deformation of the remaining portion of the supporting unit 1200. Accordingly, only the remaining portion of supporting unit 1200 except for the coupling unit 1220 may be elastically deformed, while the coupling unit 1220 is barely elastically deformed. Meanwhile, as the thickness T1 of the coupling unit 1220 is formed thicker, the rigidity of the coupling unit 1220 increases, so that the patch-attaching unit 1300 may be maintained firmly supported on the coupling unit 1220 even if an external force is exerted to the supporting unit 1200.

The coupling unit 1220 may have a fitting groove 1230. The end 1310 of the patch-attaching unit 1300 may be coupled by fitting into the fitting groove 1230. At least a portion of the fitting groove 1230 may be opened outward. That is, the fitting groove 1230 may have a shape that is open toward one surface constituting the coupling unit 1220. For example, the fitting groove 1230 may be open in the upside direction with respect to FIG. 2. In addition, the fitting groove 1230 may be opened toward at least one of the front and rear directions of the coupling unit 1220 with respect to FIG. 2. As at least a portion of the fitting groove 1230 is formed to be open outward, the end 1310 of the patch-attaching unit 1300 may be coupled by sliding in the fitting groove 1230. That is, the end 1310 of the patch-attaching unit 1300 may be inserted by sliding into the coupling unit 1220 through the opening of the fitting groove 1230. In this case, the end 1310 in the patch-attaching unit 1300 may be coercively bound to the fitting groove 1230.

The patch-attaching unit 1300 may include a bent part 1320. The bent part 1320 may be formed further inside of the patch-attaching unit 1300 than the end 1310. The bent part 1320 may be a portion formed by bending, and the end 1310 may be a portion formed outside with respect to the bent part 1320. As one example, the bent part 1320 may be bent at a right angle. In case the fitting groove 1230 is formed by opening from the upside of the coupling unit 1220, the patch-attaching unit 1300 may be provided in a form where the central portion is disposed horizontally, and the end 1310 bent from each bent part 1320 is inserted into the fitting groove 1230 downward from the upside.

Hereinafter, with particular reference to FIG. 3, the coupling unit 1220 along with another embodiment of the present disclosure will be described in detail.

According to another embodiment of the present disclosure, an end 1310 of the patch-attaching unit 1300 may include a portion more convexly formed in a preset direction than the other portion. As one example, it was shown in FIG. 3 that, when the applicator 1000 is viewed from the front, the end 1310 is formed to be convex in the extending direction of the patch-attaching unit 1300, and the end 1310 has a bulb shape as a whole. This can also be expressed as the cross-section of end 1310 is formed extruding as a first shape.

Accordingly, the fitting groove 1230 may have a shape such that the convex shape of the end 1310 may be caught by the elastic restoring force in the direction in which elastic restoring force is applied when the patch-attaching unit 1300 is elastically deformed. Accordingly, even if the patch-attaching unit 1300 is elastically deformed by the use of the applicator 1000, it is possible to prevent the end 1310 from being separated from the fitting groove 1230. As one example, the fitting groove 1230 may be concavely formed to correspond to the convex shape of the end 1310, and the end 1310 and fitting groove 1230 may be matched with each other. It can also be expressed that the cross-section of the fitting groove 1230 is formed as a second shape corresponding to the first shape of the end 1310.

In case the end 1310 of the patch-attaching unit 1300 is provided as the first shape and the fitting groove 1230 is provided as the second shape, it is possible to effectively prevent the end 1310 fitted into the fitting groove 1230 from being separated to the outside.

For example, as shown in FIG. 3, the end 1310 of the patch-attaching unit 1300 may be formed extruding outwardly formed as a first shape with cross-section convexly shaped, and correspondingly, the fitting groove 1230 may be formed as a second shape concave to correspond to the first shape. In this case, it is possible to prevent the patch-attaching unit 1300 from being separated from the coupling unit 1220 to upside even where an external force is exerted to the patch-attaching unit 1300, such as pulling the patch-attaching unit 1300 to the upside. However, the first shape and the second shape are not limited thereto, and any shape may be possible as long as it can prevent the end 1310 of the patch-attaching unit 1300 from being separated from the fitting groove 1230 to the upside.

Hereinafter, a coupling unit 1220 according to another embodiment of the present disclosure will be described in detail with reference to FIG. 4. The coupling unit 1220 according to another embodiment of the present disclosure may further have pressing blocks 1240 disposed.

The pressing blocks 1240 may be placed on both sides of the end 1310 at the fitting groove 1230. The pressing blocks 1240 may be placed inside and outside the fitting groove 1230, respectively, with the end 1310 as the center. Before the end 1310 and the pressing blocks 1240 are inserted into the fitting groove 1230, the sum of the thickness of the end 1310 and the thickness of the pressing blocks 1240 on both sides may be slightly larger than that of the inner width of the fitting groove 1230. In response to the end 1310 and the pressing blocks 1240 on both sides being inserted into the fitting groove 1230, the pressing blocks 1240 on both sides are pressed toward the end 1310, the end 1310 is pressed, and the end 1310 may be compressed and fixed. Accordingly, in response to the end 1310 and the pressing blocks 1240 on both sides are coupled to the fitting groove 1230, as the end 1310 is compressed, the end 1310 of the patch-attaching unit 1300 may be strongly fitted and coupled to the fitting groove 1230.

In this embodiment, the pressing blocks 1240 have been described as an example of being placed on both sides of the end 1310, but it may be provided on only one side, and in this case, the pressing blocks 1240 may work in the manner of pressing the end 1310 toward one inner wall of the fitting groove 1230. That is, one or more pressing blocks 1240 may be provided. In any case, the sum of the thickness of the end 1310 and the thickness of one or more pressing blocks 1240 may be formed to be larger than that of the inner width of the fitting groove 1230, thereby achieving the effect of pressing and fixing the end 1310.

Meanwhile, the cross-sectional shape of the pressing blocks 1240 and the cross-sectional shape of the fitting groove 1230 are each shown in a rectangular shape in FIG. 4, but each of cross-sectional shapes of pressing block 1240 and the fitting groove 1230 is not limited thereto.

FIG. 5 is a top plan view separately showing the appearance of a patch-attaching unit in which an adsorption groove is formed according to one embodiment of the present disclosure.

Hereinafter, the patch-attaching unit 1300 is described in detail with reference to FIG. 5. The patch-attaching unit 1300 has one or more adsorption grooves 1330 formed in the first adhesive surface S1 of the patch-attaching unit 1300.

The adsorption grooves 1330 are formed by depression in patch-attaching unit 1300. As the adsorption groove 1330 is formed by depression, in response to the patch P being deformed in the state of being in contact, negative pressure may be exerted to the patch P, and the patch P may be more strongly attached to the patch-attaching unit 1300.

In this case, the size of the adsorption grooves 1330 may be formed to be smaller than the size of the patch P. The size of the adsorption groove 1330 may be formed to be smaller than the size of the other side in the patch P. Preferably, the maximum width of the adsorption groove 1330 may be formed smaller than the minimum width of the other side of the patch P.

For example, in case both the patch P and the adsorption grooves 1330 are provided in a circular shape, the diameter of the adsorption grooves 1330 may be formed to be smaller than the diameter of the patch P. In this case, one or more adsorption grooves 1330 are placed on one patch P, and a stronger adsorption force may be exerted to the patch P. In addition, in case the patch P is provided in an oval shape and the adsorption groove 1330 is provided in a circular shape, the diameter of the adsorption groove 1330 may be formed to be smaller than the minimum width of patch P.

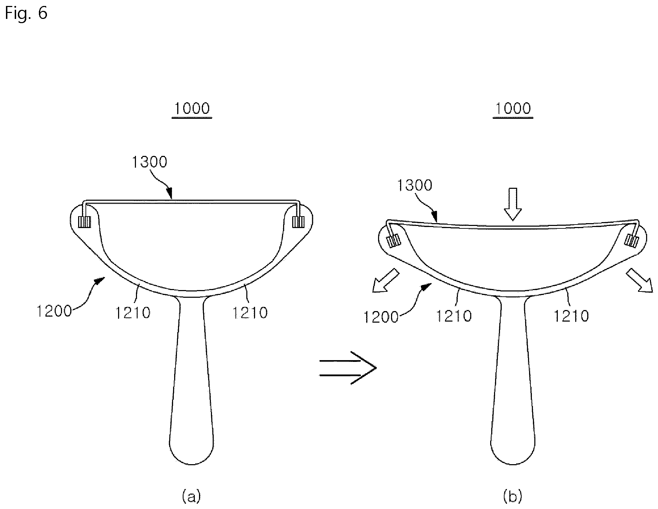

FIGS. 6A and 6B show the transformation process of the supporting unit and the patch-attaching unit according to the embodiment of the present disclosure.

Hereinafter, with reference to FIGS. 6A and 6B, before and after deforming the supporting unit 1200 and the patch-attaching unit 1300 will be compared and described in detail. FIG. 6A shows the supporting unit 1200 and the patch-attaching unit 1300 before being transformed, and FIG. 6B shows the supporting unit 1200 and patch-attaching unit 1300 each being transformed.

For example, when the patch-attaching unit 1300 is contacted and then pressed against a target site of the patch adhesive, such as the amorphous curved part of he face, the patch-attaching unit 1300 may be elastically deformed corresponding to the target site.

In addition, in response to the patch-attaching unit 1300 is brought into closer contact with the target site, the upper end side of the supporting unit 1200 may also be in contact with the target site. Herein, the upper end side of the supporting unit 1200 in contact with the target site may be elastically deformed to open outward. The supporting unit 1200, which opens outward, pulls the patch-attaching unit 1300, and at the same time, the patch-attaching unit 1300 is further pressed toward the target site side. Accordingly, the patch P can be perfectly adhered to and pressed against the amorphous target site, and the phenomenon of lifting up due to the patch P not being accurately adhered to the skin of the user may be prevented.

In addition, since the patch P may be in surface contact with the patch-attaching unit 1300 on a surface, folding during the process of being separated from the release sheet or attached to the skin of the user may be effectively prevented.

Meanwhile, the first elastic modulus E1 of the supporting unit 1200 may be provided to be greater than the second elastic modulus E2 of the patch-attaching unit 1300. In this case, when an external force of the same magnitude is exerted to the applicator 1000, amount of deformation of the patch-attaching unit 1300 is more likely to be formed greater than the amount of deformation of the supporting unit 1200. Accordingly, the patch-attaching unit 1300 may be easily deformed in response to the surface shape of the amorphous target site, while the supporting unit 1200 may provide strong support for the patch-attaching unit 1300.

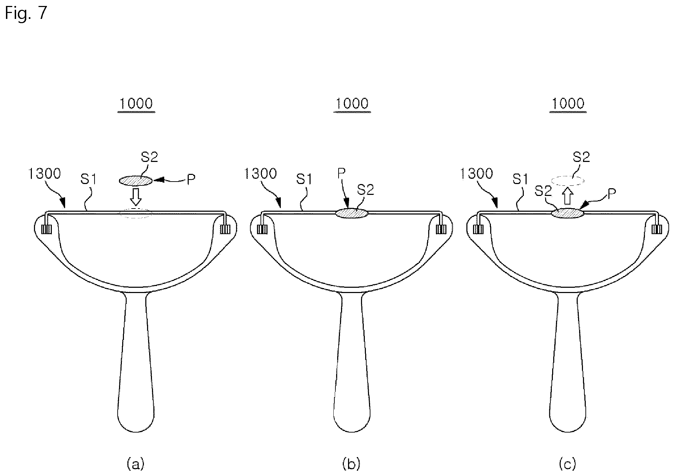

FIGS. 7A, 7B, and 7C show the process of attaching and detaching a patch to an applicator according to an embodiment of the present disclosure.

Hereinafter, referring to FIGS. 7A, 7B, and 7C, the process of attaching the patch P to the patch-attaching unit 1300 and separating the patch P from the patch-attaching unit 1300 will be described in detail.

FIG. 7A shows the process of removing the patch P and adhering it to the patch-attaching unit 1300, FIG. 7B shows the state of the patch P being adhered to the patch-attaching unit 1300, and FIG. 7C shows the process of the patch P being separated from the patch-attaching unit 1300.

The first adhesive surface S1 is provided on one side of the patch-attaching unit 1300. The second adhesive surface S2 is provided on one side of the patch P. The other side of the patch P may be attached to the first adhesive surface S1.

The patch P may be provided to a user with a plurality of pieces arranged in a member on the release sheet (not shown). The patch P may be provided in a state adhered to the release sheet. Herein, the adhesion force between the second adhesive surface S2 and the release sheet may be less than the adhesion force between the first adhesive surface S1 and the other side of the patch P. For this reason, in response to the patch P being adhered to the release sheet, the first adhesive surface S1 is brought into contact with the other side of patch P, and then in response to the applicator 1000 being separated from the release sheet, the other side of patch P may be separated from the release sheet on condition that the other side is adhered to the first adhesive surface S1. (FIG. 7A and FIG. 7B)

The adhesion force between the first adhesive surface S1 and the other side of the patch P may be provided to be less than the adhesion force between the second adhesive surface S2 and the skin.

In other words, the adhesive force exerted by the first adhesive surface S1 on patch P may be less than the adhesive force exerted by the patch P on the user's skin. For this reason, in response to the patch P being adhered to the first adhesive surface S1 is brought into contact with the skin and then in response to the applicator 1000 being separated from the skin, the patch P may be separated from the patch-attaching unit 1300 on condition that the second adhesive surface S2 of patch P is adhered to the skin. (FIG. 7C)

Since the second adhesive surface S2 of the patch P may be provided with a cosmetic material, the patch P attached to the skin of the user through the above process may effectively deliver the cosmetic material to the skin of the user.

### [Reference Signs List]

1000: Applicator
1100: Handle
1200: Supporting unit
1210: Extending unit
1220: Coupling unit
1230: Fitting groove
1240: Pressing block
1300: Patch-attaching unit
1310: End
1320: Bent part
1330: Adsorption groove
E1: First elastic modulus
E2: Second elastic modulus
P: Patch
S1: First adhesive surface
S2: Second adhesive surface

## Claims

1. An applicator (1000) for attaching a patch (P) to the skin, the applicator comprising: a supporting unit (1200), which elastically deforms in one direction and then restores in the other direction; and a patch-attaching unit (1300), supported by the supporting unit, and having a first adhesive surface (S1) formed on one side for temporarily adhering a patch, wherein the first adhesive surface has one or more adsorption grooves (1330) formed by depression.

2. The applicator of claim 1, further comprising:
a handle (1100) provided for gripping by a user,
wherein the supporting unit is connected to the handle.

3. The applicator of claim 2, wherein the supporting unit extends from the handle on both sides and has a symmetrical shape with respect to the handle.

4. The applicator of claim 1, wherein the supporting unit is formed concavely toward the patch-attaching unit in a curved shape with a preset curvature, and the curved surface is transformed into an open shape and then restored to the original state.

5. The applicator of claim 1, wherein the patch-attaching unit is provided to be elastically deformable, the patch-attaching unit is stretched together when the supporting unit is elastically deformed and stretched, and the patch-attaching unit is restored together to the original state when the supporting unit is restored to the original state.

6. The applicator of claim 1, wherein the supporting unit has a coupling unit (1220) formed to which the patch-attaching unit can be coupled and fixed.

7. The applicator of claim 6, wherein the coupling unit has a fitting groove (1230) into which the patch-attaching unit is coupled by fitting.

8. The applicator of claim 7, wherein at least a portion of the fitting groove can be opened to the outside, and the end (1310) of the patch-attaching unit is coupled by sliding with the fitting groove.

9. The applicator of claim 7, wherein an end (1310) of patch-attaching unit includes a portion more convexly formed in a preset direction than the other portion, and
the fitting groove has a shape that allows the convex shape of the end to be caught in the direction in which an elastic restoring force is exerted when the patch-attaching unit is elastically deformed.

10. The applicator of claim 7, wherein one or more pressing blocks (1240) are placed on the fitting groove, and
the pressing block is inserted into the fitting groove and presses an end of the patch-attaching unit.

11. The applicator of claim 10, wherein the sum of the thickness of the end and the thickness of pressing block is greater than the inner width of the fitting groove, and the end is pressed and fixed when the end and the pressing block are fitted into the fitting groove.

12. The applicator of claim 1, wherein the size of the adsorption groove is formed smaller than the size of the patch.

13. The applicator of claim 1, wherein the supporting unit has a first elastic modulus (E1), the patch-attaching unit has a second elastic modulus (E2), the supporting unit and the patch-attaching unit are provided to be elastically deformable, respectively, and
wherein the first elastic modulus is greater than the second elastic modulus.

14. The applicator of claim 1, wherein an adhesive force exerted by the first adhesive surface on the patch is less than an adhesive force exerted by the patch on the skin of the user, and
the adhesive force exerted by the first adhesive surface on the patch is greater than the adhesive force between the patch and the release sheet in a state that the patch is adhered to the release sheet.

## Patentansprüche

1. Applikator (1000) zum Anbringen eines Pflasters (P) auf der Haut, wobei der Applikator Folgendes umfasst:
eine unterstützende Einheit (1200), die sich in einer Richtung elastisch verformt und dann in der anderen Richtung wiederherstellt, und
eine Einheit zum Anbringen eines Pflasters (1300), die von der unterstützenden Einheit getragen wird und die eine erste Klebefläche (S1) aufweist, die auf einer Seite ausgebildet ist, um ein Pflaster vorübergehend zum Haften zu bringen,
wobei die erste Klebefläche eine oder mehrere durch eine Vertiefung gebildete Adsorptionsmulden (1330) aufweist.

2. Applikator gemäß Anspruch 1, der des Weiteren Folgendes umfasst:
einen Griff (1100), der zum Greifen durch einen Benutzer vorgesehen ist,
wobei die unterstützende Einheit mit dem Griff verbunden ist.

3. Applikator gemäß Anspruch 2, wobei sich die unterstützende Einheit auf beiden Seiten von dem Griff aus erstreckt und eine symmetrische Form in Bezug auf den Griff aufweist.

4. Applikator gemäß Anspruch 1, wobei die unterstützende Einheit konkav in Richtung der Einheit zum Anbringen eines Pflasters in einer gekrümmten Form mit einer vorgegebenen Krümmung ausgebildet ist und die gekrümmte Oberfläche in eine offene Form überführt und anschließend in den ursprünglichen Zustand zurückversetzt wird.

5. Applikator gemäß Anspruch 1, wobei die Einheit zum Anbringen eines Pflasters elastisch verformbar vorgesehen ist, die Einheit zum Anbringen eines Pflasters, wenn die unterstützende Einheit elastisch verformt und gedehnt wird, zusammen damit gedehnt wird, und die Einheit zum Anbringen eines Pflasters, wenn die unterstützende Einheit in den ursprünglichen Zustand zurückversetzt wird, zusammen damit in den ursprünglichen Zustand zurückversetzt wird.

6. Applikator gemäß Anspruch 1, wobei die unterstützende Einheit eine ausgeformte Kopplungseinheit (1220), an der die Einheit zum Anbringen eines Pflasters angekoppelt und befestigt sein kann, aufweist.

7. Applikator gemäß Anspruch 6, wobei die Kopplungseinheit eine Einpassnut (1230) aufweist, in die die Einheit zum Anbringen eines Pflasters durch Einpassen eingekoppelt ist.

8. Applikator gemäß Anspruch 7, wobei mindestens ein Abschnitt der Einpassnut nach außen geöffnet sein kann und das Ende (1310) der Einheit zum Anbringen eines Pflasters durch Einschieben mit der Einpassnut gekoppelt ist.

9. Applikator gemäß Anspruch 7, wobei ein Ende (1310) der Einheit zum Anbringen eines Pflasters einen Abschnitt aufweist, der in einer vorgegebenen Richtung stärker konvex ausgeformt ist als der andere Abschnitt, und
die Einpassnut eine Form aufweist, die es ermöglicht, dass die konvexe Form des Endes in der Richtung ergriffen wird, in der eine elastische Rückstellkraft ausgeübt wird, wenn die Einheit zum Anbringen eines Pflasters elastisch verformt wird.

10. Applikator gemäß Anspruch 7, wobei ein oder mehrere Pressblöcke (1240) an der Einpassnut angeordnet sind und
der Pressblock in die Einpassnut eingeführt ist und auf ein Ende der Einheit zum Anbringen eines Pflasters Druck ausübt.

11. Applikator gemäß Anspruch 10, wobei die Summe aus der Dicke des Endes und der Dicke des Pressblocks größer als die innere Breite der Einpassnut ist und
das Ende gepresst und fixiert ist, wenn das Ende und der Pressblock in die Einpassnut eingepasst sind.

12. Applikator gemäß Anspruch 1, wobei die Größe der Adsorptionsmulde kleiner als die Größe des Pflasters ausgebildet ist.

13. Applikator gemäß Anspruch 1, wobei die unterstützende Einheit einen ersten Elastizitätsmodul (E1) aufweist, die Einheit zum Anbringen eines Pflasters einen zweiten Elastizitätsmodul (E2) aufweist, die unterstützende Einheit und die Einheit zum Anbringen eines Pflasters jeweils so ausgebildet sind, dass sie elastisch verformbar sind, und
wobei der erste Elastizitätsmodul größer als der zweite Elastizitätsmodul ist.

14. Applikator gemäß Anspruch 1, wobei eine von der ersten Klebefläche auf das Pflaster ausgeübte Haftkraft geringer als eine vom Pflaster auf die Haut des Benutzers ausgeübte Haftkraft ist, und
in einem Zustand, in dem das Pflaster an der Trennfolie haftet, die Haftkraft, die von der ersten Klebefläche auf das Pflaster ausgeübt wird, größer als die Haftkraft zwischen dem Pflaster und der Trennfolie ist.

## Revendications

1. Applicateur (1000) pour fixer un patch (P) à la peau, l'applicateur comprenant :
une unité de support (1200), qui se déforme élastiquement dans une direction et
puis se rétablit dans l'autre direction ; et
une unité de fixation du patch (1300) supportée par l'unité de support et ayant une première surface adhésive (S1) formée sur un côté pour faire coller temporairement un patch,
la première surface adhésive comportant une ou plusieurs rainures d'adsorption (1330) formées par dépression.

2. Applicateur selon la revendication 1, comprenant en outre :
une poignée (1100) fournie pour la préhension par l'utilisateur,
l'unité de support étant connectée à la poignée.

3. Applicateur selon la revendication 2, dans lequel l'unité de support s'étend de la poignée sur les deux côtés et a une forme symétrique par rapport à la poignée.

4. Applicateur selon la revendication 1, dans lequel l'unité de support est formée de manière concave vers l'unité de fixation du patch dans une forme incurvée avec une courbure préréglée et la surface incurvée est transformée en une forme ouverte puis rétablie à l'état original.

5. Applicateur selon la revendication 1, dans lequel l'unité de fixation du patch est fournie pour être élastiquement déformable, l'unité de fixation du patch est étirée ensemble quand l'unité de support est déformée élastiquement et étirée et l'unité de fixation du patch est rétablie ensemble à l'état original quand l'unité de support est rétablie à l'état original.

6. Applicateur selon la revendication 1, dans lequel l'unité de support comporte une unité de couplage (1220) formée à laquelle l'unité de fixation du patch peut être couplée et fixée.

7. Applicateur selon la revendication 6, dans lequel l'unité de couplage comporte une rainure d'ajustement (1230) dans laquelle l'unité de fixation du patch est couplée par ajustement.

8. Applicateur selon la revendication 7, dans lequel au moins une partie de la rainure d'ajustement peut être ouverte à l'extérieur et l'extrémité (1310) de l'unité de fixation du patch est couplée par coulissage avec la rainure d'ajustement.

9. Applicateur selon la revendication 7, dans lequel une extrémité (1310) de l'unité de fixation du patch comprend une partie formée de manière plus convexe dans une direction préréglée que l'autre partie et
la rainure d'ajustement a une forme qui permet à la forme convexe de l'extrémité d'être prise dans la direction dans laquelle une force de rappel élastique est exercée quand l'unité de fixation du patch est déformée élastiquement.

10. Applicateur selon la revendication 7, dans lequel un ou plusieurs blocs de pressage (1240) sont placés sur la rainure d'ajustement et
le bloc de pressage est inséré dans la rainure d'ajustement et presse une extrémité de l'unité de fixation du patch.

11. Applicateur selon la revendication 10, dans lequel la somme de l'épaisseur de l'extrémité et de l'épaisseur du bloc de pressage est plus grande que la largeur intérieure de la rainure d'ajustement et
l'extrémité est pressée et fixée quand l'extrémité et le bloc de pressage sont ajustés dans la rainure d'ajustement.

12. Applicateur selon la revendication 1, dans lequel la taille de la rainure d'adsorption est formée plus petite que la taille du patch.

13. Applicateur selon la revendication 1, dans lequel l'unité de support possède un premier module élastique (E1), l'unité de fixation du patch possède un second module élastique (E2), l'unité de support et l'unité de fixation du patch sont fournies pour être élastiquement déformables, respectivement, et
le premier module élastique étant plus grand que le second module élastique.

14. Applicateur selon la revendication 1, dans lequel une force adhésive exercée par la première surface adhésive sur le patch est inférieure à une force adhésive exercée par le patch sur la peau de l'utilisateur et
la force adhésive exercée par la première surface adhésive sur le patch est plus grande que la force adhésive entre le patch et la feuille de détachement dans un état où le patch est collé à la feuille de détachement.
